# FASCICULE DE BREVET EUROPEEN

(11) **EP 4 360 595 B1**
(45) Date de publication et mention de la délivrance du brevet: **20.05.2026**
(21) Numéro de dépôt: 23205713.3
(22) Date de dépôt: 25.10.2023
(51) Int. Cl.: A61F 2/40, A61F 2/32, A61F 2/38

(54) **IMPLANT ARTICULAIRE EN CÉRAMIQUE POURVU DE MOYENS DE VERROUILLAGE POUR SON VISSAGE**
KERAMISCHES GELENKIMPLANTAT MIT VERRIEGELUNGSMITTELN ZUM VERSCHRAUBEN DESSELBEN
CERAMIC JOINT IMPLANT WITH LOCKING MEANS FOR ITS SCREWING

(30) Priorité: 27.10.2022 FR 2211231
(43) Date de publication de la demande: 01.05.2024
(73) Titulaire: FX Shoulder Solutions, 01440 Viriat (FR)
(72) Inventeur: DAUDET, Jean-Marie, 01000 Bourg-en-Bresse (FR)
(74) Mandataire: LLR

(56) Documents cités:
- EP-A1- 0 884 032
- EP-A1- 2 689 750
- FR-A1- 2 802 799

## Description

L'invention concerne un implant de prothèse articulaire. Plus spécifiquement, l'invention concerne un implant de prothèse articulaire dont la surface externe articulaire est en céramique.

On connaît déjà dans l'état la technique, des prothèses articulaires de plusieurs types, par exemple des prothèses d'épaule, des prothèses de hanche ou encore des prothèses de genou.

L'ensemble de ces prothèses articulaires comprennent deux implants présentant chacun une surface externe articulaire destinée à venir en contact l'une avec l'autre, reproduisant ainsi l'articulation. Afin de reproduire la meilleure qualité d'articulation possible, il est connu d'utiliser pour ces surfaces externes articulaires en contact, des couples de matériaux favorables, ces couples pouvant être deux matériaux dits « dur » ou un matériau « dur » et un matériau dit « mou ». Les matériaux « durs » connus et utilisés dans les implants de prothèse articulaires sont l'alliage de Cobalt Chrome, alliage de titane acier inox ou encore la céramique. Les matériaux dits « mous » peuvent être en plastique, par exemple en polyéthylène de masse molaire très élevée (UHMWPE) ou en polyétheréthercétone (PEEK) ou en PEKK(poly ether ketone ketone) et leurs dérivés.

Le Cobalt Chrome est un alliage particulièrement apprécié des fabricants d'implants de prothèse articulaire car il présente des propriétés tribologiques et mécaniques exceptionnelles qui permettait notamment de visser et serrer l'implant concerné (à l'aide d'une ou plusieurs vis localisées dans l'implant) soit directement dans l'os du patient, soit dans un élément lui-même fixé à l'os du patient (par exemple une métaglène pour une prothèse totale d'épaule inversée), et ce, sans risque de détérioration de l'implant.

Cependant, les dernières normes en vigueur (et notamment la norme mise à jour du règlement délégué (UE) 2020/217 du 4 octobre 2019 REACH) tendent à interdire l'utilisation du chrome cobalt comme matériau pour les implants de prothèse articulaire au motif que le cobalt est considéré comme une substance potentiellement cancérigène, mutagène ou toxique pour le patient. Dès lors, il convient d'envisager des matériaux durs alternatifs.

Un matériau de type dur connu est la céramique. Ce matériau présente l'avantage d'être biocompatible et particulièrement adapté pour les frottements au regard de ses propriétés de dureté. Cependant, la céramique est un matériau très peu ductile et les contraintes mécaniques très fortes et localisées résultantes du serrage précité au moyen d'une ou plusieurs vis risquent d'engendrer des fissures au sein de la céramique, voire même une rupture de cette dernière. Un tel risque n'est pas acceptable que ce soit pour le chirurgien lors de l'opération ou pour le patient qui risque de devoir remplacer sa prothèse trop rapidement. De plus, il n'est actuellement pas autorisé de remettre un implant en céramique après une rupture de la céramique d'un premier implant. Il est dès lors obligatoire de s'orienter vers un autre couple de matériaux.

Pour remédier à ces inconvénients, on connaît déjà dans l'état la technique, des implants de prothèse articulaire comprenant un élément rapporté par clippage entre une glénosphère, dont la surface externe articulaire est en céramique, et une vis de serrage destinée à être introduite par serrage dans l'os du patient (ou dans une métaglène intermédiaire lorsque l'os du patient est une glène). Cet élément rapporté permet de répartir les contraintes mécaniques issues du serrage de la vis de serrage, de manière homogène au sein de la glénosphère en céramique de sorte que celle-ci ne se fissure ou ne se casse pas. Cependant, de telles solutions ne sont pas satisfaisantes puisqu'elles ne permettent pas de garantir la bonne tenue dudit élément rapporté, que ce soit lors du serrage de la vis de serrage ou dans le temps, après l'opération. En effet, il a été constaté que cet élément rapporté, sous l'action des contraintes locales appliquées par la tête de vis lors du serrage de cette dernière, se déforme fortement ce qui engendre son dé-clipsage avec la glénosphère. Dès lors, ces solutions ne permettent pas de garantir une répartition homogène des contraintes mécaniques résultantes du serrage au sein de la glénosphère en céramique qui risque de se fissurer ou de se casser lors de l'opération ou très rapidement après celle-ci. En outre, après l'opération et durant la vie de la prothèse, cet élément rapporté subit également des contraintes locales pouvant engendrer un désagrément similaire, entrainant des conséquences identiques pour l'intégrité de la glénosphère en céramique.

Le document EP 2 689 750 A1 divulgue un implant selon le préambule de la revendication 1.

L'invention a notamment pour but de permettre l'utilisation d'un tel implant en céramique pourvu d'un élément rapporté qui surmonte l'ensemble des inconvénients précités.

A cet effet l'invention a pour objet un implant comprenant :
- un corps principal principalement en céramique et comprenant une surface externe articulaire en céramique de forme concave ou convexe,
- au moins une vis de serrage configurée pour permettre le serrage du corps principal sur l'os du patient ou sur un élément intermédiaire fixé audit os,
- au moins une bague rapportée dans le corps principal et formant avec ce dernier une chambre emprisonnant la tête de la vis de serrage et comprenant une surface d'appui formant butée pour ladite tête de la vis de serrage, la bague rapportée étant configurée pour être en contact avec la vis de serrage durant ledit serrage, la bague rapportée (4) comprenant un orifice central traversé par la vis de serrage, la bague rapportée étant configurée pour répartir les contraintes mécaniques issues du serrage de la vis de serrage de manière homogène au sein du corps principal, la bague rapportée comprenant un premier organe formant la surface externe de la bague rapportée et comprenant des moyens de clipsage au corps principal et un deuxième organe formant entretoise entre le premier organe et la vis de serrage, ledit deuxième organe étant assemblé au sein du premier organe, formant ladite surface d'appui et définissant l'orifice central, le premier organe et le deuxième organe étant deux organes distincts l'un de l'autre et comprenant des moyens de verrouillage configurés pour interdire toute désolidarisation entre eux après assemblage.

Ainsi, il est possible de réaliser un implant pour prothèse articulaire en céramique (ou principalement en céramique) qui soit à la fois fonctionnel et robuste et qui ne risque pas de fissurer ou de casser lors du serrage de la vis de serrage (ou après l'opération). En effet, l'implant de prothèse articulaire selon l'invention permet de réduire les contraintes mécaniques transmises au corps principal, issues du serrage de la vis de serrage, par rapport aux solutions de l'art antérieur, grâce notamment à l'assemblage entre le premier organe et le deuxième organe de la bague rapportée. Comme les contraintes transmises *in fine* au corps principal de la glénosphère, par le premier organe de la bague rapportée, sont réparties de manière homogène au sein du corps principal, on réduit ainsi drastiquement les risques de fissure ou de cassure du corps principal en céramique, que ce soit lors de l'opération de serrage de la vis de serrage ou après l'opération. Par « répartition homogène des contraintes mécaniques issues du serrage de la vis », on entend que les contraintes sont réparties sur toute la surface du corps principal en contact avec la bague rapportée (plus précisément avec son premier organe), ce qui permet ainsi d'éviter que ne s'applique sur le corps principal en céramique une contrainte qui serait supérieure à la limite élastique et qui entrainerait une rupture de la céramique. Grace à une grande surface de contact radiale du premier organe de la bague rapportée avec le corps principal, il est possible d'avoir une répartition des forces sur une surface plus importante du corps principal pour que les contraintes localisées soient les plus faibles possibles.

Le deuxième organe permet en outre d'empêcher toute déformation du premier organe lors du vissage de la vis de serrage dans l'os du patient ou dans l'élément intermédiaire fixé audit os. Ainsi, tout dé-clipsage entre les moyens de clipsage du premier organe de la bague rapportée et le corps principal de la glénosphère est empêché, ce qui sécurise davantage l'utilisation de l'implant.

En outre, les moyens de verrouillage de l'assemblage entre le premier organe et le deuxième organe interdisent toute désolidarisation entre eux après assemblage, tant lors du vissage de la vis de serrage dans l'os du patient ou dans l'élément intermédiaire fixé audit os qu'avant l'opération (par exemple à cause de vibrations transmises à la bague rapportée durant le transport de l'implant) ou qu'après l'opération (par exemple à cause de vibrations résultant des activités du patient). Ainsi, cela optimise la robustesse et la bonne tenue dans le temps de l'implant.

La bague rapportée (et donc le premier organe et/ou le deuxième organe) peut être réalisée en tout matériaux permettant une bonne répartition des contraintes mécaniques au sein du corps principal. Le premier organe et/ou le deuxième organe peuvent notamment être réalisés tout ou partie en métal, en plastique ou en céramique. Elle peut également être réalisée avec plusieurs matériaux différents dont une composition des matériaux précités.

Suivant d'autres caractéristiques optionnelles de l'implant de prothèse articulaire selon l'invention prises seules ou en combinaison :
- le premier organe comprend une portion taraudée et le deuxième organe comprend une portion filetée correspondante, la portion filetée et la portion taraudée étant configurées pour permettre de visser le deuxième organe avec le premier organe, de préférence, le sens du vissage du deuxième organe avec le premier organe étant inversé à celui réalisé lors serrage de la vis de serrage,
- la portion taraudée et la portion filetée présentent un pas à gauche ou un pas à droite, de préférence compris entre 0.5mm et 2mm, la portion filetée représentant de préférence être un filet métrique, un filet d'artillerie ou un filet carré,
- les moyens de verrouillage comprennent une gorge circulaire et une collerette configurée pour s'engager dans ladite gorge, la gorge étant formée sur le premier organe ou sur le deuxième organe et la collerette étant respectivement formée sur le deuxième organe ou le premier organe,
- les moyens de verrouillage comprennent au moins un clip et au moins un orifice configuré pour être traversé par le clip, le clip étant formé sur le premier organe ou sur le deuxième organe et l'orifice étant respectivement formé sur le deuxième organe ou le premier organe,
- le premier organe comprend des moyens périphériques déformables élastiquement,
- le corps principal est une glénosphère, la vis de serrage étant configurée pour permettre le serrage entre la glénosphère et une métaglène fixée sur la glène du patient,
- la glénosphère a un diamètre compris entre 30 et 46mm, et/ou
- le corps principal est un plateau tibial céramique, un condyle de genou, une glène anatomique, un cotyle de hanche ou une cupule céramique.

L'invention a également pour objet une prothèse articulaire comprenant un implant selon l'une quelconque des variantes précédentes.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue en coupe d'un implant de prothèse articulaire selon l'invention,
[Fig. 2] la figure 2 est une vue en coupe de l'implant de la figure 1 assemblé à une métaglène,
[Fig. 3] la figure 3 est une vue en perspective (figure 3A), de dessus (figure 3B) et en couple (3C) d'une bague rapportée telle que présente dans l'implant des figures 1 et 2,
[Fig. 4] la figure 4 est une vue en perspective (figure 4A), de dessus (figure 4B) et en couple (4C) d'un premier organe de la bague rapportée de la figure 4,
[Fig. 5] la figure 5 est une vue en perspective (figure 5A), de dessus (figure 5B) et en couple (5C) d'un deuxième organe de la bague rapportée de la figure 4.

### Description détaillée

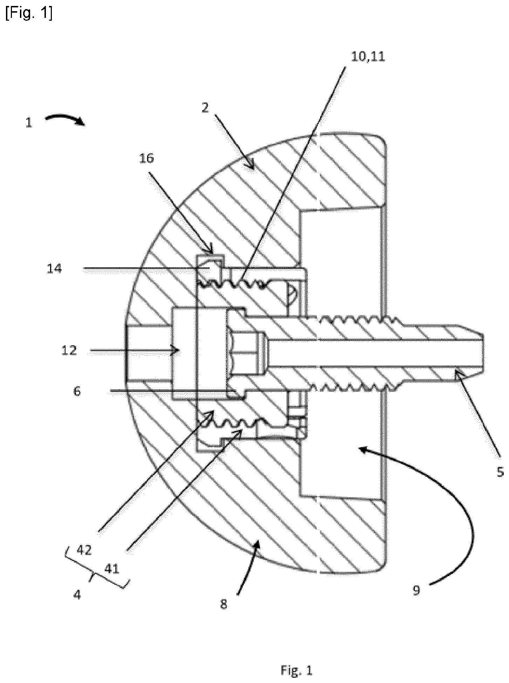

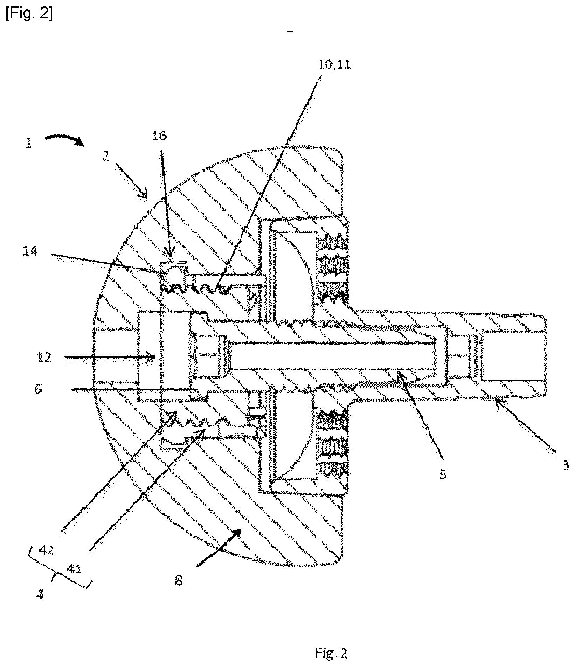

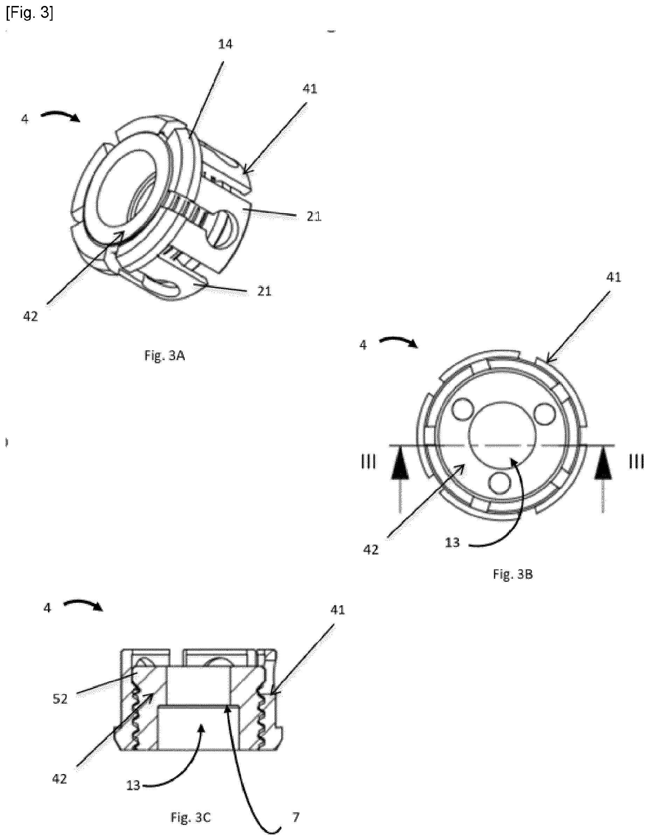

On a représenté sur les figures 1 et 2 un implant de prothèse articulaire selon un premier mode de réalisation de l'invention, désigné par la référence générale 1. Sur les figures 3A à 5C, on a représenté une bague rapportée, désignée par la référence générale 4 ou l'un de ses deux organes constitutifs. Il convient de noter que les éléments analogues entre les figures sont désignés par des références identiques.

L'implant de prothèse articulaire 1 ainsi décrit comprend :
- un corps principal 2 principalement en céramique et comprenant une surface externe articulaire 8 en céramique de forme concave ou convexe,
- au moins une vis de serrage 5 configurée pour permettre le serrage du corps principal 2 sur l'os du patient ou sur un élément intermédiaire 3 fixé audit os,
- au moins une bague rapportée 4 dans le corps principal 2 et formant avec ce dernier une chambre 12 emprisonnant la tête 6 de la vis de serrage 5 et comprenant une surface d'appui 7 formant butée pour ladite tête 6 de la vis de serrage 5, la bague rapportée 4 étant configurée pour être en contact avec la vis de serrage 5 durant ledit serrage, la bague rapportée 4 comprenant un orifice central 13 traversé par la vis de serrage 5, la bague rapportée 4 étant configurée pour répartir les contraintes mécaniques issues du serrage de la vis de serrage 5 de manière homogène au sein du corps principal 2. La bague rapportée 4 comprend un premier organe 41 formant la surface externe de la bague rapportée 4 et comprenant des moyens de clipsage 14 au corps principal 2 et un deuxième organe 42 formant une entretoise entre le premier organe 41 et la vis de serrage 5, ledit deuxième organe 42 étant assemblé au sein du premier organe 41, formant ladite surface d'appui 7 et définissant l'orifice central 13, le premier organe 41 et le deuxième organe 42 étant deux organes distincts l'un de l'autre et comprenant des moyens de verrouillage 51, 52 configurés pour interdire toute désolidarisation entre eux après assemblage.

Dans le mode de réalisation décrit à l'appui des figures, le corps principal 2 correspond à une glénosphère 2 dont la vis de serrage 5 permet le serrage avec un élément intermédiaire 3 fixé à l'os, en l'occurrence la métaglène 3.

La glénosphère 2 en céramique peut avoir un diamètre allant de 30 à 46mm, peut être centrée ou excentrée et peut être latéralisée le cas échéant.

La glénosphère 2 et la métaglène 3 sont assemblées l'une à l'autre par l'intermédiaire de la vis de serrage 5. Afin d'assurer un assemblage encore meilleur, un cône morse (non représenté) est prévu entre la glénosphère 2 et la métaglène 3.

La glénosphère 2 comprend un logement 9 dimensionné pour recevoir la bague rapportée 4 et formé avec cette dernière la chambre 12 emprisonnant la tête 6 de la vis de serrage 5.

L'implant de prothèse articulaire décrit correspond donc à un implant glénoïdien de prothèse d'épaule inversée. Néanmoins, les caractéristiques décrites dans le cadre de ce mode de réalisation peuvent s'appliquer à toute prothèse comprenant un implant articulaire présentant une surface articulaire en céramique et étant vissé et serré au moyen d'au moins une vis de serrage, sur l'os du patient ou sur un élément intermédiaire fixé audit os. A titre d'exemple, l'invention concerne également des implants de prothèses de hanche ou de genou.

Le type de céramique utilisé pour le corps principal peut être tout type de céramique connu de l'homme du métier. A titre d'exemple, il peut s'agir de l'alumine, de la zircone ou d'un matériau composite. La céramique étant un matériau de type dur, il peut être utilisé dans le cadre de couple de matériaux « dur/dur » ou « dur-mou ». Dans ce second exemple, le matériau dit « mou » peut être du polyéthylène de masse molaire très élevée (UHMWPE), du polyétheréthercétone (PEEK), du PEKK(poly ether ketone ketone) ou leurs dérivés

Comme précisé précédemment, la bague rapportée 4 est clipsée au sein du logement 9 de la glénosphère 2, via des moyens de clipsage, présents partiellement sur son premier organe 41, et forme ainsi, avec le logement 9, la chambre 12 emprisonnant la tête 6 de la vis de serrage 5. Plus précisément, les moyens de clipsage se matérialisent par des dents présentes aux extrémités de moyens périphériques élastiquement déformables (prenant la forme d'ailettes 21), formant ensemble une nervure 14, et par une rainure circulaire 16 formée au sein du logement 9 de la glénosphère 2 et dont la forme permet de recevoir et bloquer la nervure 14 formée par les dents, de sorte à permettre le clipsage du premier organe 41 de la bague rapportée 4. Plus précisément, l'élasticité conférée au premier organe 41 par ses ailettes 21 élastiquement déformables permet de le déformer suffisamment pour introduire la nervure 14 au sein de la rainure 16. Lorsque les ailettes 21 reprennent leur forme initiale, la nervure 14 se retrouve coincer dans la rainure 16, engendrant ainsi le clipsage du premier organe 41 au sein du logement 9 de la glénosphère 2. Ainsi, il existe une surface importante de contact entre le premier organe 41 et la glénosphère 2, à savoir toute la zone de contact radiale entre la nervure 14 et la rainure 16. Il est ainsi possible de répartir de manière homogène sur l'ensemble de cette large surface de contact entre le logement 9 de la glénosphère 2 et la bague rapportée 4 et donc, *in fine,* de minimiser la force des contraintes subies localement par la glénosphère 2 en céramique, ce qui réduit le risque de fissures ou de cassure de cette dernière.

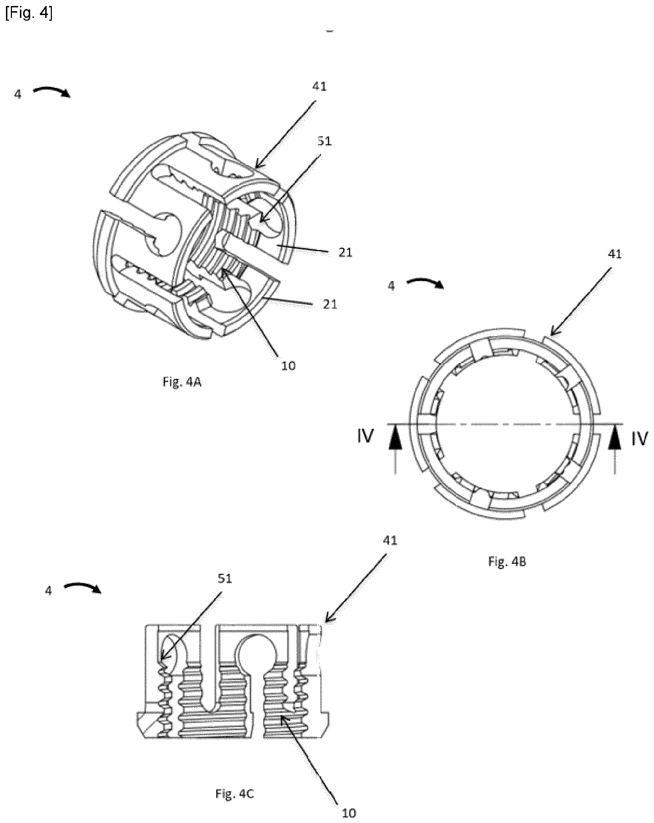

Comme déjà mentionné, la bague rapportée 4 comprend un premier organe 41 (visible aux figures 4A à 4C). Ce premier organe assure principalement la fonction d'assemblage à la glénosphère 2. Dans le mode de réalisation décrit, cet assemblage est réalisé par clipsage du premier organe 41 dans le logement 9 de la glénosphère 2. Pour ce faire, le premier organe est pourvu des dents présentes aux extrémités de moyens périphériques élastiquement déformables (sous la forme d'ailettes 21), formant ensemble la nervure 14. Ces ailettes 21 sont fendues (comme cela est particulièrement visible aux figures 3A, 4A et 4C) afin de conférer au premier organe 41 les propriétés élastiques suffisante à son clipsage au sein de la rainure 16.

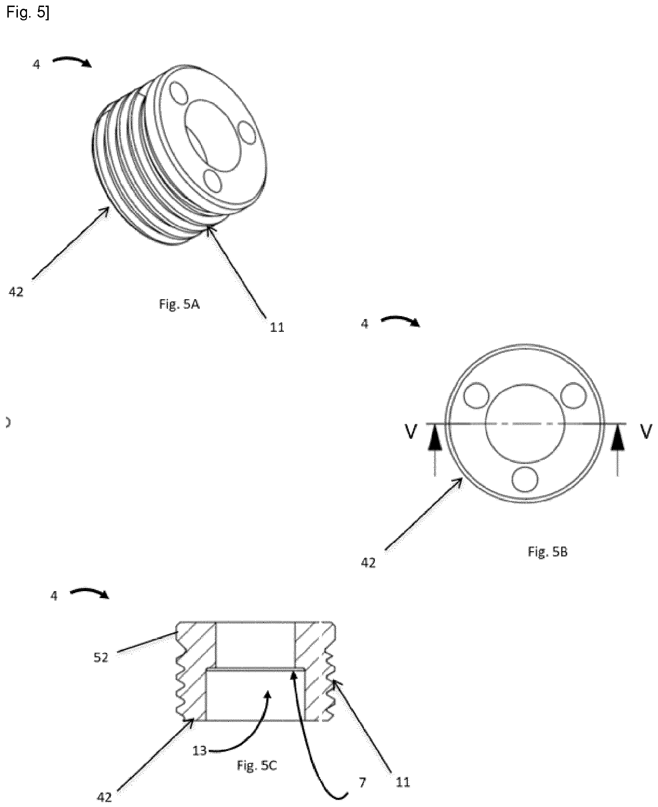

La bague comprend en outre un deuxième organe 42 dont le positionnement et la forme permettent de contraindre le premier organe 41 dans sa position clipsée, notamment lors du serrage de la vis de serrage 5. Ce deuxième organe 42 correspond à une entretoise entre le premier organe et la vis de serrage 5 et assure donc le rôle de verrou pour le premier organe 41. Ce deuxième organe 42 (figures 5A à 5C), de forme cylindrique, présente un diamètre inférieur à celui du premier organe 41, ce qui permet de l'assembler au sein du premier organe 41. Dans le mode de réalisation décrit, cet assemblage est réalisé par vissage. Pour ce faire, le deuxième organe 42 comprend une portion filetée 11 radiale et externe de forme complémentaire à la portion taraudée 10 radiale interne du premier organe 41. Avantageusement, le vissage du deuxième organe 42 avec le premier organe 41 est inversé à celui réalisé lors serrage de la vis de serrage 5. Autrement dit, le sens du vissage entre deuxième organe 42 et le premier organe 41 est inversé par rapport au sens selon lequel la glénosphère va être vissée soit dans l'os du patient, soit dans la métaglène 3. Ainsi, on réduit les risques de dévissage entre le premier organe 41 et le deuxième organe 42 lors de l'étape de serrage de la vis de serrage 5. Avantageusement, la portion taraudée 10 et la portion filetée 11 présentent un pas à gauche ou un pas à droite. En outre, la portion filetée 11 représente de préférence être un filet métrique, un filet d'artillerie ou un filet carré. Enfin, le pas de la portion taraudée 10 et la portion filetée 11 peut être standardisé et/ou un pas fin. En ce sens, le pas est avantageusement compris entre 0.5mm et 2mm ce qui permet d'avoir un pas très fin.

Le deuxième organe 42 comprend une collerette 52 à son extrémité distale qui, lorsque le deuxième organe 42 est en position finale au sein du premier organe 41 (c'est-à-dire une fois qu'il est vissé complètement), s'engage dans la gorge circulaire 51 prévue dans le premier organe 41. Ainsi cette position finale entre le premier organe 41 et le deuxième organe 42 est verrouillée de telle sorte que toute désolidarisation entre eux est rendue impossible. Dans une variante non représentée, ces moyens de verrouillage (à savoir la collerette et la gorge) pourraient être respectivement prévus sur le premier organe et sur le deuxième organe. Selon une autre variante non représentée de l'invention, les moyens de verrouillage peuvent, alternativement ou cumulativement, comprendre au moins un clip et au moins un orifice configuré pour être traversé par le clip permettant également de verrouiller l'assemblage dans la position finale entre le premier organe et le deuxième organe de la bague rapportée. Ce clip et cet orifice peuvent être respectivement prévus sur le premier organe et sur le deuxième organe et inversement. Afin de verrouiller avec une plus grande force de verrouillage, les moyens de verrouillage peuvent comprendre plusieurs clips et plusieurs orifices traversant dédiées et/ou plusieurs collerettes et plusieurs gorges dédiées.

Par ailleurs, ce deuxième organe 42 comprend un lamage interne qui forme ladite surface d'appui 7 (figures 3A à 3C) pour la tête de vis 6 et présente un orifice traversant qui définit l'orifice central 13 traversé par la vis de serrage 5. Le deuxième organe 42 comprend donc une surface d'appui 7 formant butée pour la tête 6 de la vis 5 lors du serrage de cette dernière.

La bague rapportée 4 comprend une zone importante de contact entre le premier organe 41 et le deuxième organe 42, à savoir toute la surface de contact entre le filet 11 du deuxième organe et le taraud 10 du premier organe 41. Ainsi, lors du vissage de la vis de serrage 5, des contraintes s'appliquent sur le deuxième organe 42 qui, de par sa configuration plus rigide que le premier organe 41, en assume une large partie et n'en transmet qu'une partie moins importante. Cette portion moins importante est répartie de manière homogène au premier organe 41 via la zone de contact entre le taraud 10 et le filet 11, puis la partie résultante est elle-même transmise à la face interne du logement 9 de la glénosphère 2 en céramique, principalement via le contact radial entre la nervure 14 et la rainure 16, mais également via le contact direct entre les ailettes déformables 21 et la face interne du logement 9 de la glénosphère 2. Il en résulte une transmission moins importante et une répartition plus homogène des contraintes issues du serrage de la glénosphère 2 sur la métaglène 3, ce qui participe *in fine* à une réduction de la force des contraintes subies localement par la glénosphère 2 en céramique, réduisant le risque de fissures ou de cassure de cette dernière.

Comme déjà mentionné, l'invention n'est pas limitée aux modes de réalisation présentés. En effet et comme évoqué précédemment, l'invention peut être appliquée à tout type d'implant articulaire présentant une surface articulaire en céramique et étant vissé et serré au moyen d'au moins une vis de serrage, sur l'os du patient ou sur un élément intermédiaire fixé audit os. A titre d'exemple, l'invention concerne également des implants de prothèses de hanche ou de genou.

### Liste de références

1 : implant
2 : corps principal/glénosphère
3 : métaglène
4 : bague rapportée
5 : vis de serrage
6 : tête de la vis de serrage
7 : surface d'appui
8 : surface externe articulaire
9 : logement du corps principal
10 : portion taraudée/taraud
11 : portion filetée/filet
12 : chambre
13 : orifice central de la bague
14 : nervure
16 : rainure
21 : ailettes déformables élastiquement
41 : premier organe de la bague rapportée
42 : deuxième organe de la bague rapportée
51 : gorge
52 : collerette

## Revendications

1. Implant de prothèse articulaire (1) comprenant :
- un corps principal (2) principalement en céramique et comprenant une surface externe articulaire (8) en céramique de forme concave ou convexe,
- au moins une vis de serrage (5) configurée pour permettre le serrage du corps principal (2) sur l'os du patient ou sur un élément intermédiaire (3) fixé audit os,
- au moins une bague rapportée (4) dans le corps principal (2) et formant avec ce dernier une chambre (12) emprisonnant la tête (6) de la vis de serrage (5) et comprenant une surface d'appui (7) formant butée pour ladite tête (6) de la vis de serrage (5), la bague rapportée (4) étant configurée pour être en contact avec la vis de serrage (5) durant ledit serrage, la bague rapportée (4) comprenant un orifice central (13) traversé par la vis de serrage (5), la bague rapportée (4) étant configurée pour répartir les contraintes mécaniques issues du serrage de la vis de serrage (5) de manière homogène au sein du corps principal (2), **caractérisé en ce que** la bague rapportée (4) comprend un premier organe (41) formant la surface externe de la bague rapportée (4) et comprenant des moyens de clipsage (14) au corps principal (2) et un deuxième organe (42) formant entretoise entre le premier organe (41) et la vis de serrage, ledit deuxième organe (42) étant assemblé au sein du premier organe (41), formant ladite surface d'appui (7) et définissant l'orifice central (13), le premier organe (41) et le deuxième organe (42) étant deux organes distincts l'un de l'autre et comprenant des moyens de verrouillage (51, 52) configurés pour interdire toute désolidarisation entre eux après assemblage.

2. Implant (1) selon la revendication précédente, dans lequel le premier organe (41) comprend une portion taraudée (10) et le deuxième organe (42) comprend une portion filetée (11) correspondante, la portion filetée (11) et la portion taraudée (10) étant configurées pour permettre de visser le deuxième organe (42) avec le premier organe (41), de préférence, le sens du vissage du deuxième organe (42) avec le premier organe (41) étant inversé à celui réalisé lors serrage de la vis de serrage (5).

3. Implant (1) selon la revendication précédente, dans lequel la portion taraudée (10) et la portion filetée (11) présentent un pas à gauche ou un pas à droite, de préférence compris entre 0.5mm et 2mm, la portion filetée (11) représentant de préférence être un filet métrique, un filet d'artillerie ou un filet carré.

4. Implant (1) selon l'une des revendications précédentes, dans lequel les moyens de verrouillage comprennent une gorge circulaire (51) et une collerette (52) configurée pour s'engager dans ladite gorge (51), la gorge (51) étant formée sur le premier organe (41) ou sur le deuxième organe (42) et la collerette (52) étant respectivement formée sur le deuxième organe (42) ou le premier organe (41).

5. Implant (1) selon l'une des revendications précédentes, dans lequel les moyens de verrouillage comprennent au moins un clip et au moins un orifice configuré pour être traversé par le clip, le clip étant formé sur le premier organe (41) ou sur le deuxième organe (42) et l'orifice étant respectivement formé sur le deuxième organe (42) ou le premier organe (41).

6. Implant (1) selon l'une des revendications précédentes, dans lequel le premier organe (41) comprend des moyens périphériques déformables élastiquement (21).

7. Implant (1) selon l'une des revendications précédentes, dans lequel le corps principal (2) est une glénosphère (2), la vis de serrage (5) étant configurée pour permettre le serrage entre la glénosphère (2) et une métaglène (3) fixée sur la glène du patient.

8. Implant (1) selon la revendication précédente, dans lequel la glénosphère (2) a un diamètre compris entre 30 et 46mm.

9. Implant (1) selon l'une des revendications 1 à 6, dans lequel le corps principal (2) est un plateau tibial céramique, un condyle de genou, une glène anatomique, un cotyle de hanche ou une cupule céramique.

10. Prothèse articulaire comprenant un implant (1) selon l'une quelconque des revendications précédentes.

## Patentansprüche

1. Implantat (1) für Gelenkprothese, umfassend:
- einen Hauptkörper (2) hauptsächlich aus Keramik, der eine Gelenkaußenfläche (8) aus Keramik konkaver oder konvexer Form umfasst,
- mindestens eine Spannschraube (5), die ausgebildet ist, um das Festspannen des Hauptkörpers (2) auf dem Knochen des Patienten oder auf einem Zwischenelement (3), das an dem Knochen befestigt ist, zu gestatten,
- mindestens einen angesetzten Ring (4) in dem Hauptkörper (2), der mit diesem eine Kammer (12) bildet, die den Kopf (6) der Spannschraube (5) einklemmt und eine Auflagefläche (7) umfasst, die einen Anschlag für den Kopf (6) der Spannschraube (5) bildet, wobei der angesetzte Ring (4) ausgebildet ist, um während des Festspannens mit der Spannschraube (5) in Kontakt zu sein, wobei der angesetzte Ring (4) eine mittige Öffnung (13) umfasst, die von der Spannschraube (5) durchquert ist, wobei der angesetzte Ring (4) ausgebildet ist, um die durch das Anziehen der Spannschraube (5) bewirkten mechanischen Kräfte gleichmäßig im Inneren des Hauptkörpers (2) zu verteilen, **dadurch gekennzeichnet, dass** der angesetzte Ring (4) ein erstes Glied (41), das die Außenfläche des angesetzten Rings (4) bildet und Einrastmittel (14) zum Einrasten an dem Hauptkörper (2) umfasst, und ein zweites Glied (42), das ein Distanzstück zwischen dem ersten Glied (41) und der Spannschraube bildet, umfasst, wobei das zweite Glied (42) in dem ersten Glied (41) montiert ist, die Auflagefläche (7) bildet und die mittige Öffnung (13) definiert, wobei das erste Glied (41) und das zweite Glied (42) zwei voneinander verschiedene Glieder sind und Verriegelungsmittel (51, 52) umfassen, die ausgebildet sind, um jedwedes Lösen voneinander nach der Montage zu verhindern.

2. Implantat (1) nach dem vorhergehenden Anspruch, wobei das erste Glied (41) einen mit Innengewinde versehenen Teil (10) umfasst und das zweite Glied (42) einen korrespondierenden mit Außengewinde versehenen Teil (11) umfasst, wobei der mit Außengewinde versehene Teil (11) und der mit Innengewinde versehene Teil (10) ausgebildet sind, um das Verschrauben des zweiten Glieds (42) mit dem ersten Glied (41) zu gestatten, wobei bevorzugt die Richtung für das Verschrauben des zweiten Glieds (42) mit dem ersten Glied (41) umgekehrt zu der ist, die beim Anziehen der Spannschraube (5) realisiert wird.

3. Implantat (1) nach dem vorhergehenden Anspruch, wobei der mit Innengewinde versehene Teil (10) und der mit Außengewinde versehene Teil (11) ein Linksgewinde oder ein Rechtsgewinde aufweisen, das bevorzugt zwischen 0,5 mm und 2 mm beträgt, wobei der mit Außengewinde versehene Teil (11) bevorzugt ein metrisches Gewinde, ein Artillerie-Gewinde oder ein Flachgewinde aufweist.

4. Implantat (1) nach einem der vorhergehenden Ansprüche, wobei die Verriegelungsmittel eine kreisförmige Nut (51) und eine Feder (52) umfassen, die dafür ausgebildet ist, in der Nut (51) in Eingriff zu kommen, wobei die Nut (51) an dem ersten Glied (41) oder an dem zweiten Glied (42) gebildet ist, und wobei die Feder (52) an dem zweiten Glied (42) bzw. dem ersten Glied (41) gebildet ist.

5. Implantat (1) nach einem der vorhergehenden Ansprüche, wobei die Verriegelungsmittel mindestens einen Clip und mindestens eine Öffnung umfassen, die dafür ausgebildet ist, von dem Clip durchquert zu werden, wobei der Clip an dem ersten Glied (41) oder an dem zweiten Glied (42) gebildet ist, und wobei die Öffnung an dem zweiten Glied (42) bzw. dem ersten Glied (41) gebildet ist.

6. Implantat (1) nach einem der vorhergehenden Ansprüche, wobei das erste Glied (41) elastisch verformbare Umfangsmittel (21) umfasst.

7. Implantat (1) nach einem der vorhergehenden Ansprüche, wobei der Hauptkörper (2) eine Glenosphäre (2) ist, wobei die Spannschraube (5) ausgebildet ist, um das Festspannen zwischen der Glenosphäre (2) und einer Glenoidbasisplatte (3), die auf der Schulterpfanne des Patienten befestigt ist, zu gestatten.

8. Implantat (1) nach dem vorhergehenden Anspruch, wobei die Glenosphäre (2) einen Durchmesser zwischen 30 und 46 mm hat.

9. Implantat (1) nach einem der Ansprüche 1 bis 6, wobei der Hauptkörper (2) ein Keramik-Tibiaplateau, ein Kniegelenksknorren, eine Schultergelenkpfanne, eine Hüftgelenkpfanne oder eine Keramik-Hüftpfanne ist.

10. Gelenkprothese, umfassend ein Implantat (1) nach einem der vorhergehenden Ansprüche.

## Claims

1. Prosthesis joint implant (1) comprising:
- a main body (2) made mainly of ceramic and comprising a joint outer surface (8) made of ceramic, of concave or convex shape,
- at least one tightening screw (5) configured to tighten the main body (4) on the patient's bone or on an intermediate element attached to said bone,
- at least one added ring (4) in the main body (2) and forming with it a chamber (12) trapping the head (6) of the tightening screw (5) and comprising a bearing surface (7) forming a stop for said head (6) of the tightening screw (5), the added ring (4) being configured to be in contact with the tightening screw (5) during said tightening, the added ring (4) comprising a central hole (14) crossed by the tightening screw (5), the added ring (4) being configured to homogeneously distribute in the main body (2) the mechanical stresses caused by tightening the tightening screw (5), **characterized in that** the added ring (4) comprises a first member (41) forming the outer surface of the added ring (4) and comprising means for clipping (14) to the main body (2) and a second member (42) acting as a spacer between the first member (41) and the tightening screw (5), said second member (42) being assembled in the first member (41), forming said bearing surface (7) and defining the central hole (13), the first member (41) and the second member (42) being two members separate from each other and comprising locking means (51, 52) configured to prevent them from separating from each other after assembly.

2. Implant (1) according to the preceding claim, wherein the first member (41) comprises a tapped portion (10) and the second member (42) comprises a corresponding threaded portion (11), the threaded portion (11) and the tapped portion (10) being configured so that the second member (42) can be screwed to the first member (41), preferably, the second member (42) being screwed to the first member (41) in the direction opposite to that when tightening the tightening screw (5).

3. Implant (1) according to the preceding claim 2, wherein the tapped portion (10) and the threaded portion (11) have a left hand pitch or a right hand pitch, preferably of between 0.5 mm and 2 mm, the threaded portion (11) preferably being a metric thread, a buttress thread or a square thread.

4. Implant (1) according to any of the preceding claims, wherein the locking means comprise a circular channel (51) and a collar (52) configured to engage in said channel (51), the channel (51) being formed on the first member (41) or on the second member (42) and the collar (52) being respectively formed on the second member (42) or the first member (41).

5. Implant (1) according to any of the preceding claims, wherein the locking means comprise at least one clip and at least one hole configured to be crossed by the clip, the clip being formed on the first member (41) or on the second member (42) and the hole being respectively formed on the second member (42) or the first member (41).

6. Implant (1) according to any of the preceding claims, wherein the first member (41) comprises elastically deformable peripheral means (21).

7. Implant (1) according to any of the preceding claims, wherein the main body (2) is a glenosphere, the tightening screw (5) being configured to tighten the glenosphere (2) onto a metaglene (3) attached to the patient's glenoid.

8. Implant (1) according to the preceding claim, wherein the diameter of the glenosphere (2) is between 30 and 46 mm.

9. Implant (1) according to claims 1 to 6, wherein the main body (2) is a ceramic tibial plateau, a knee condyle, an anatomic glenoid, a hip acetabulum or a ceramic cup.

10. Joint prosthesis comprising an implant (1) according to any of the preceding claims.
